# EUROPEAN PATENT APPLICATION

(11) **EP 4 154 871 A1**
(43) Date of publication of application: **29.03.2023**
(21) Application number: 21198775.5
(22) Date of filing: 24.09.2021
(51) Int. Cl.: A61K 9/16

(54) **PROCESS FOR GENERATING MICROPARTICLES**

(71) Applicant: Bayer AG, 51373 Leverkusen (DE)
(72) Inventor: DOBROWOLSKI, Adrian., 42899 Remscheid (DE); MAGGIONI, Giovanni Maria., 50676 Köln (DE); SOWA, Michal., 42109 Wuppertal (DE); BOTHE, Clemens., 51375 Leverkusen (DE); ESSER, Eva., 40233 Duesseldorf (DE); NÜBOLDT, Christoph., 50969 Köln (DE)
(74) Representative: BIP Patents

(57) **Abstract**

What is described herein relates to a method for generating micro- particles comprising the steps of
a) providing a homogenous supersaturated solution of a substance in a crystallization medium
b) providing a seeding material characterized by a nano-particle size of a d90 between 10 nm and 999 nm comprising at least one stabilizer, wherein said seeding material is of the same substance as the substance of the homogenous supersaturated solution of step a)
c) bringing the homogenous supersaturated solution in contact with the seeding material,
optionally obtaining micro-particles.

## Description

The present invention relates to a nano-seeding process.

A significant proportion of active ingredients e.g. of active pharmaceutical ingredients and active agrochemical ingredients have poor solubility and/or required a high bioavailability and short dissolution times. To improve the poor solubility especially in water and optimize high bioavailability and short dissolution times, a commonly accepted method is to reduce the particle size of the active ingredients to create a large surface-to-volume ratio and hence a high solubility particle interface.

A well-known method to produce microparticles is dry-milling. However, this method has some limitations, such as high energy consumption, low yields, formation of encrustations, and difficult-to-control particle sizes and surface properties, which complicate microparticle production and consistent quality. Therefore, there is a need for an improved process for generating micro-particles.

This need is met by a simplified process for generating micro-particles.

According to a first aspect what is described herein relates to a process for generating micro- particles comprising the steps of
a) providing a homogenous supersaturated solution of a substance in a crystallization medium
b) providing a seeding material characterized by a nano-particle size of a d90 between 10 nm and 999 nm comprising at least one stabilizer, wherein said seeding material is of the same substance as the substance of the homogenous supersaturated solution of step a)
c) bringing the homogenous supersaturated solution in contact with the seeding material,
d) optionally obtaining micro-particles.

According to a second aspect what is described herein relates to the use of nanoparticles of a d90 between 10 nm and 999 nm as seeding material in cooling crystallization.

### BRIEF DESCRIPTION OF THE DRAWINGS

### FIGURES

FIG. 1 shows that using nano seeding material leads to micro-particles comparable to micro-particles generated with conventional processes.
FIG. 2 depicts that less nano-seeding material is required to obtain a similar particle size distribution of the generated microparticles as with conventional processes.
FIG. 3 shows that using nano seeding material (different active ingredient than the active ingredient of Fig. 1) leads to micro-particles comparable to micro-particles generated with conventional processes.
FIG. 4 shows that the type of nano-seeding material (suspension, powder or as tablet) does not play a key role in the crystallization process.

### DETAILED DESCRIPTION

### DEFINITIONS

As used herein, the term "particle" refers to a solid, gel, or semisolid material having a relatively small size.

As used herein the term "micro-particle" which is used synonymously with microparticle refers to particles having an average particle size of between ≥ 999 nm to 100µm.

Preferably the micro-particles obtained with the process for generating micro-particles described herein have an average d90 of between 2µm and 50µm, more preferably between 2µm and 30µm, most preferably between 2µm and 20µm.

As used herein the term "nano" refers to particles having an average particle size - i.e. d90 of the distribution - of ≥ 10 nm to ≤ 999 nm.

Herein the nanoparticles (also termed nano-particles) have a particle size distribution of d90 of ≥ 10 nm to ≤ 999 nm preferably ≥ 10 nm to < 400 nm especially preferred ≥ 10 nm to < 200 nm. The particle size distribution can be determined by methods known in the art such as laser diffraction or Dynamic Light Scattering (DLS). The term d50 or d90 means that 50% or 90% of the total product volume consists of particles smaller than the given value for d50 or d90. The given particle size corresponds to the hydrodynamic particle size when determined by DLS (dynamic light scattering) or XRD (X-Ray diffraction) and for both methods an equivalent size for spheres is given and meant here.

As used herein the term "seeding material" refers to nano-particles of the same substance or composition as the micro-particles to be generated by the process described herein. In general the seeding material is analyzed for its quality, purity, and particle size distribution. The quality of the seeding material i.e. the polymorphism and/or pseudo-polymorphism can be determined, e.g. by means of X-ray diffraction and/or Raman and/or IR spectroscopy. The size distribution of the seeding material can be determined by laser diffraction and/or dynamic light scattering. The purity of the seeding material can be determined e.g. by chromatography.

Homogenous as used herein refers to the fact that a material is uniform in appearance. When referring to a solution homogenous therefore means that no distinct solid and liquid phases co-exist.

When referring to a suspension - e.g. after the homogenous supersaturated solution and the seeding material have been brought into contact - "homogenous" is herein meant to be if no spontaneous segregation of particles according to their size can be detected within the suspension and/or if no dead volumes, i.e. portion of reactor where the flow stagnates, can be observed, e.g. by means of visual analysis or tracer-based methods, or predicted, e.g. by means of CFD simulations within 30 min to 2 days after preparation of the solution. The selection of the relevant operating conditions to ensure good miscibility can be assessed by calculating the relevant non-dimensional numbers (e.g. Newton, Reynold, Peclet), which account for the geometric properties of the chosen vessel and impeller. The correlations between such numbers and the relevant operating conditions are tabulated in the relevant literature, e.g. Perry Chemical Engineering Handbook, 8th Edition, McGraw-Hill, 2007.

A skilled person is aware of the fact that upon spontaneous formation of particles, or after their addition through seeding, the supersaturated solution becomes a suspension, i.e. a system where distinct solid and liquid phases co-exist.

As used herein the term "supersaturated" refers to a solution, in which the concentration of a solute exceeds the concentration of its thermodynamically equilibrium value.

The supersaturated homogenous solution used in the process described herein is in the metastable zone width. Metastable zone width as used herein refers to the interval of temperature and concentration in which a solution is supersaturated, but kinetically stable, i.e. the spontaneous appearance and growth of crystals whilst thermodynamically possible, is not observed. In other words, a skilled person is aware of the fact that the metastable zone width refers to the combination of temperature range and concentration range i.e. the zone in which a solution of a given solute is supersaturated but does not yet spontaneously crystallize and is not supersaturated any longer i.e. the addition of seeding material would no longer result in crystal formation. The metastable zone width i.e. the temperature and concentration values for a given solute in a given crystallization medium have to be determined on a case by case basis. A person skilled in the art knows how to perform the required experiments for a given solute in a given crystallization medium. For example, a skilled person would perform a screening of solvents compatibly with chemical constraints such as product degradation followed by screening for the possible presence of (pseudo)polymorphs and measuring the relevant solubilities. Alternatively, computer models can also be exploited to a certain degree to mitigate (but not fully replace) in vitro experiments, e.g. by performing DFT or MD simulations to assess the relative stability of different polymorphs, or by estimating the solubility, e.g. using PC-SAFT and the related theory of groups.

As used herein the term "flowability" refers to powder flow i.e. the capacity to move by flow of loose particulate solids.

A person skilled in the art is aware of the fact that the flowability of a powder depends on various factors such as particle size distribution, particle shape, chemical composition of the particles, moisture and temperature. A Schulze ring shear tester can be used for comparing flowabilities of different powders.

As used herein the term "stabilizer" refers to a polymer that sterically and/or a surfactant that electrostatically or sterically stabilizes the nano-particles.

As used herein the term "active ingredient" refers to an agent, active ingredient, compound, substance, compositions, or mixtures thereof, that provides a pharmacological and/or agrochemical effect.

As used herein the term "crystallization medium" refers to a liquid in which the substance constituting the nano-particles and the to be generated micro-particles is soluble in and from which the substance can crystallize when the medium is supersaturated. A skilled person is aware of the fact that in cases of where more than one active ingredient is present in a given composition e.g. active ingredient combinations in step a) the substance to be crystallized is present as the homogenous supersaturated solution while possible other (active) ingredients in the crystallization medium are present as suspension.

A person skilled in the art knows how to determine a suitable crystallization medium depending on the specific process as well as chemical and regulatory requirements. Standard crystallization media for pharmaceutical and agro-chemical relevant substances are, for example, water, methanol, ethanol, acetone, acetic acid, depending on whether an inorganic (water) or organic (other) medium is required, whether the solubility is promoted by protonated (water, alcohols, acids) or non-protonated (acetone or esters) solvents and/or by the presence of H-bonds and/or other coordination groups. In one example described herein the micro-particles were active ingredient micro-particles and were crystallized in acetic acid, where solubility strongly depends on temperature and where the formation of other undesired polymorphs does not take place at the process conditions. In the case of acetylsalicylic acid as active ingredient a mixture of acetic anhydride and acetic acid is used as crystallization medium, due to the requirements of synthesis occurring prior to crystallization. Another known crystallization medium is ethanol.

### DETAILED DESCRIPTION

As specified above according to a first aspect what is described herein relates to a process for generating micro-particles comprising the steps of
a) providing a homogenous supersaturated solution of a substance in a crystallization medium
b) providing a seeding material characterized by a nano-particle size of a d90 between 10 nm and 999 nm comprising at least one stabilizer, wherein said seeding material is of the same substance as the substance of the homogenous supersaturated solution of step a)
c) bringing the homogenous supersaturated solution in contact with the seeding material,
d) optionally obtaining micro-particles.

The improved process - also termed nano-seeding - for generating microparticles described herein is a shorter, faster, less tedious, less time consuming and less error prone process as it omits the need to perform a micronization step in order to generate homogenous microparticles of a suitable d90 value. This has the advantage that challenges such as caking during micronization of cohesive powder active ingredients (API) which complicates process control due to clogging are no longer of relevance.

Moreover, the improved process for generating microparticles described herein is also more cost effective since the omitted micronization is an energy-intensive process carried out under high pressures (up to 7 bar nitrogen or more) and low loadings.

In addition, the improved process for generating microparticles gives comparable yields to the methods described in the art for generating microparticles, i.e. it does not suffer the draw-back of other processes omitting micronization such as early termination of the cooling crystallization that show a significant loss of yield. In the nano-seeding process, on the contrary, the final crystallization conditions, e.g. the final temperature can be kept. Consequently, the final yield related to the solubility is also unchanged, which would not occur if early termination was implemented.

Furthermore, the use of nano particles as seeding material increases the likelihood that no long needles will be formed during crystallization, or will be formed to a lesser extent. This can be advantageous in many ways. Needle-shaped powders generally have poorer flowability than spherical or cuboidal powders. In addition, the filterability of these particles from the mother liquor after crystallization may well be improved. The processability of the end product is thus improved.

In a preferred embodiment of the method described herein the seeding material is provided at a concentration between 0,5 wt% to 30 wt%, preferably 0,9 wt% to 3wt%, even more preferably 1 wt% to 2 wt% - wherein the weight percentage (wt%) is given with respect to total amount of the same substance the seeding material is composed of in the homogenous supersaturated solution of step a) .

The seeding material can be obtained by the method as described in WO 2021/069350. Said seeding material can be provided as powder or as tablet.

If the provided seeding material is obtained by the methods as described in WO 2021/069350, the seeding material is obtained via
A) suspending particles in an aqueous solution of a polymer;
B) drying the mixture obtained after step A);
characterized in that
in step A), the d90 value of the particle size distribution is ≤ 1 µm, and in that prior to step B), the particles are contacted with an ionic surfactant
and wherein the particles and the polymer are present in a weight ratio of ≥ 1:2 to ≤ 5:1 to each other. Preferably if the provided seeding material is obtained by this methods the polymer and the surfactant are present in a weight ratio of ≥ 10:1 to ≤ 300:1 to each other.

Preferably, the nano-particles which are suspended in step A) of the method described in WO 2021/069350 and provided in step b) of the method described herein, respectively, are obtained via nano-grinding thereby providing a seeding material characterized by a nano-particle size of a d90 between 10 nm and 999 nm comprising at least one stabilizer.

Suitable methods of nano-grinding (also termed nano grinding, nanogrinding or nanomilling) can be selected from the group consisting of wet bead milling in stirred media mills, wet bead milling in planetary mills - especially suitable for small amounts- and high pressure homogenization. Alternatively, the application of high shear forces in aqueous suspensions like in a high pressure homogenization process or the application of high impact forces between the particles like in a microfluidizer can be used to produce nano-particles.

To a skilled person it is clear that if nano grinding is used for providing the seeding material said nano grinding can only be performed if the nano-particles are not completely dissolved. Hence the skilled person would choose a suitable temperature within the temperature range of between 253 K and 323 K, preferably between 273 K and 303 K for nano-grinding the nano-particles in the aqueous suspension

Preferably, if the nano-particles which are suspended in step A) of the method described in WO 2021/069350 are obtained in the form of particles by means of nano-grinding, a preferred nano-grinding time is observed during grinding in order to achieve better redispersion of the dried powder containing nanoparticles. This preferred grinding time (t-preferred) is significantly longer than the grinding time that would typically be required to achieve the necessary particle size. Preferably said preferred grinding time (t-preferred) is at least 1.5 times t0, preferably it is at least 2 times t0 and particularly preferably at least 4 times t0. Here, t0 is considered to be the usual grinding time at which the d90 value of the particle size distribution is 1.5 times the d90 value reached after 12 hours and is referred to as d90(12h). In other words, this means that d90 (12h) is 2/3 of the d90 value at the usual grinding time t0. Grinding time is understood to mean the residence time of the suspension in the mill, which explicitly means that the possible residence time of the suspension in an optionally present holding tank is not counted towards the grinding time, i.e. towards the comminution time (tz).

If the nano-particles provided in step b) of the method described herein, are provided suspended in a crystallization medium and obtained via nano-grinding, the grinding time is coupled to the d90 of the grinded suspension. The grinding can be stopped at once if the desired particle size is reached. Said nano-grinded seeding material comprising at least one stabilizer suspended in a crystallization medium can be used directly in step c) of the method described herein or can be stored. If the nano-grinded seeding material comprising at least one stabilizer suspended in a crystallization medium is stored it is preferably kept in the fridge at temperatures below 10°C. To enhance the stability of the particles, the nano suspensions can be dried. In this state the solid has to be stored 40°C below the glass transition temperature of the used polymer.

The at least one stabilizer may be selected from the group: Alkyl celluloses, hydroxyalkyl celluloses, hydroxyalkylalkyl celluloses, carboxyalkyl celluloses, alkali metal salts of carboxyalkyl celluloses, carboxyalkylalkyl celluloses, carboxyalkyl cellulose esters, starches, pectins, chitin derivatives, polysaccharides, polyacrylic acid and its salts, polymethacrylic acid and its salts, polyvinyl alcohol, polyvinyl pyrrolidone, polyalkylene oxides, or a mixture of at least two of the above polymers.

Preferably, the polymer is selected from methylcellulose, hydroxymethylcellulose, hydroxyethylcellulose, hydroxypropylcellulose, hydroxybutylcellulose, hydroxyethylmethylcellulose, hydroxypropylmethylcellulose, carboxymethylcellulose, sodiumcarboxymethylcellulose, carboxymethylethylcellulose, carboxyalkylcellulose ester, starches, sodium carboxymethylamylopectin, chitosan, dextran sulfate sodium salt, alginic acid, alkali metal and ammonium salts of alginic acid, carrageenans, galactomannans, tragacanth, agar-agar, gum arabic, guar gum, xanthan gum, polyacrylic acid and its salts, polymethacrylic acid and its salts, polyvinyl alcohol, polyvinylpyrrolidone, polyethylene oxide, polypropylene oxide, copolymers of ethylene oxide and propylene oxide, N-vinylpyrrolidone-vinyl acetate copolymers or a mixture of at least two of the aforementioned polymers. Polyvinylpyrrolidones (especially K12 and K30 types) and N-vinylpyrrolidone-vinyl acetate copolymers are particularly preferred. Preferably, the polymer can be a copolymer of ethylene oxide and propylene oxide, in particular a poloxamer, or a polyvinylpyrrolidone, in particular PVPK30.

The ionic surfactant may be an anionic, cationic or zwitterionic (amphoteric) surfactant.

Employing an ionic surfactant has the advantage that it further stabilizes the nano-particles in the seeding material electrostatically,

The ionic surfactant is selected from acylamino acids (and salts thereof), such as: acylglutamates, for example sodium acylglutamate, di-TEA-palmitoylaspartate and sodium acaprylglutamate; acylpeptides, for example palmitoyl hydrolyzed milk protein, sodium cocoyl hydrolyzed soy protein and sodium/potassium cocoyl hydrolyzed collagen; Sarcosinates, for example, myristoyl sarcosine, TEA-lauroyl sarcosinate, sodium lauroyl sarcosinate and sodium cocoyl sarcosinate; Taurates, for example, sodium lauroyl taurate and sodium methyl cocoyl taurate; acyl lactylates, luroyl lactylate, caproyl lactylate, alaninates; carboxylic acids and derivatives, such as: carboxylic acids, for example lauric acid, aluminum stearate, magnesium alkanolate and zinc undecylenate; ester carboxylic acids, for example calcium stearoyl lactylate and sodium PEG lauramide carboxylate; Ether carboxylic acids, for example sodium laureth carboxylate and sodium PEG cocamide carboxylate; phosphoric acid esters and salts, such as DEA-oleth-phosphate and dilaureth-phosphate; sulfonic acids and salts, such as acyl-isethionates, e.g. sodium/ammonium cocoyl isethionate, alkyl aryl sulfonates, alkyl sulfonates, for example sodium coco monoglyceride sulfate, sodium C-olefin sulfonate, sodium lauryl sulfoacetate and magnesium PEG cocamide sulfate, sulfosuccinates, for example dioctyl sodium sulfosuccinate, disodium laureth sulfosuccinate, disodium lauryl sulfosuccinate and disodium undecylenamido MEA sulfosuccinate; as well as sulfuric acid esters, such as alkyl ether sulfate, for example sodium, ammonium, magnesium, MIPA, TIPA laureth sulfate, sodium myreth sulfate and sodium C-pareth sulfate, alkyl sulfates, for example sodium, ammonium and TEA lauryl sulfate.

The ionic surfactant(s) may further be selected from the group of cationic surfactants. Cationic surfactants to be advantageously used are alkylamines, alkylimidazoles, ethoxylated amines, quaternary surfactants and esterquats.

Quaternary surfactants contain at least one N atom covalently bonded to 4 alkyl or aryl groups. This leads, independent of the pH value, to a positive charge. Advantageously alkyl betaine, alkyl amidopropyl betaine and alkyl amidopropyl hydroxysulfaine are used. The cationic surfactants used can further preferably be selected from the group of quaternary ammonium compounds, in particular benzyltrialkylammonium chlorides or bromides, such as benzyldimethylstearylammonium chloride, further alkyltrialkylammonium salts, for example cetyltrimethylammonium chloride or bromide, alkyldimethylhydroxyethylammonium chlorides or bromides, dialkyldimethylammonium chlorides or bromides, alkylamidethyltrimethylammonium ether salts, alkylpyridinium salts, for example lauryl- or cetylpyrimidinium chloride, imidazoline derivatives and compounds of cationic character such as amine oxides, for example alkyldimethylamine oxides or alkylaminoethyldimethylamine oxides. Advantageously, cetyltrimethylammonium salts are to be used in particular.

The ionic surfactant or surfactants can be selected from the group of amphoteric surfactants.

Advantageous amphoteric surfactants to be used are: Acyl/dialkyl ethylenediamines, for example sodium acylamphoacetate, disodium acylamphodipropionate, disodium alkylamphodiacetate, sodium acylamphohydroxypropyl sultonate, disodium acylamphodiacetate and sodium acylamphopropionate, and N-alkylamino acids, for example, aminopropylalkylglutamide, alkylaminopropionic acid, natuium alkylimidodipropionate and lauroamphocarboxyglycinate.

Preferred surfactants include sodium dodecyl sulfate (SDS), sodium docusate (dioctyl sodium sulfosuccinate), sodium oleate and/or sodium deoxycholate.

Preferably the ionic surfactant is present in an amount of between 0.001 wt% and 10 wt% preferably 0.001 wt% and 0.4 wt%, most preferably between 0.01 wt% and 0.2 wt% in suspension.

Preferably the at least one stabilizer is used in a ratio of 1:1 to 10:1 (nanoparticle to stabilizer) preferably in a ratio of 10:3. A skilled person knows whether to use only one or more than one stabilizer in a given situation.

A preferred combination of polymer and surfactant are hydroxypropylmethylcellulose as polymer and sodium dodecyl sulfate as surfactant.

Prior to optional step d) of the process described above the generated microparticles are present in the crystallization medium and said the suspension is herein referred to composition comprising microparticles.

In a preferred embodiment of the process described herein the substance provided as homogenous supersaturated solution is an active ingredient and hence the nano-particles and any generated micro-particles are active ingredient particles.

In an embodiment of step c) of bringing the homogenous supersaturated solution in contact with the seeding material occurs at a stirring rate of between 10rpm to 600 rpm, more preferably of between 100rpm to 500 rpm, even more preferably of between 300 to 400rpm.

In another embodiment step c) of bringing the homogenous supersaturated solution in contact with the seeding material occurs at a temperature of between 30°C and 120°C, preferably 40°C and 100 °C, even more preferably at between 45°C and 92°C.

In a further embodiment after bringing the homogenous supersaturated solution in contact with the seeding material in step c) the solution is left ripening for between 10min to 60 min, preferably for between 20 to 50 min most preferably for 30 min.

In a further preferred embodiment of the process described herein after bringing the homogenous supersaturated solution in contact with the seeding material the supersaturated solution is cooled thereby causing cooling crystallization.

The cooling parameters such as the cooling rate, the cooling time and the end point depend on a given process. A person skilled in the art knows how to define the optimal cooling rate using the solubility curve of a given homogenous supersaturated solution to determine how fast the solution has to be cooled, and for how long, to generate the optimal particle size distribution, maximize the final yields, and minimize the overall crystallization time, e.g. as detailed in the Crystallization Technology Handbook, Mersmann, CRC Press 2001.

Technical constraints that are taken into account by a skilled person in determining the cooling parameters are e.g. the maximum duty of the heat exchanger, together with further chemical, economical and/or planning considerations - such as maximum process time and product degradation. To find the optimal cooling parameters to be used for a given process under the given (equipment) conditions the residual supersaturation at the end of the process is measured for different cooling rates and different overall process times to determine the loss of yield with respect to the maximum theoretical value. Moreover, also the following parameters are measured with the method specified above to determine the optimal feasible cooling rate: final particle size distribution preferably together with polymorphic purity, filterability as well as chemical purity. As people skilled in the art know, the final particle size distribution affects the filterability of the product, which must therefore be also assessed. A standard practice to determine filterability is to measure the filter-cake resistance as expressed by the so-called "alpha-value". Further details can be found, for instance, in Filters and Filtration Handbook, 6th edition, Trevor and Chase, Butterworth-Heinemann 2015.

Hence due to practical and process constraints the typical time of cooling crystallization will be between 0,5h and 8h, preferably between 2h and 5h, most preferably around 3h whereas the typical cooling rate will be between 0,05 °C/minute and 10°C/minute preferably between 0.1 °C/minute and 0.3 °C/minute. In some cases, the cooling rate might not be constant but faster or slower during the process.

Devices for carrying cooling crystallizations are known in the art and are for example a curved impeller or Rush turbines, possibly in combination with baffles to increase the efficiency of mixing.

The stirring rate is adjusted for the specific mixer chosen, in order to maintain an equivalent specific energy input. The relevant nondimensional numbers, such as the Newton number, dependent on the type of mixer and on the presence/absences of baffles can be found by a person skilled in the art in the relevant published tables (e.g. in Perry Chemical Engineering Handbook, 8th Edition, McGraw-Hill, 2007).

In a preferred embodiment a curved impeller is used for the cooling crystallization. The specific energy input should be between 0.01 and 0.4 W/kg, preferably between 0.03 and 0.35 W/kg, most preferably 0.05 and 0.3 W/kg.

Preferably the stirring rate during cooling crystallization is the same as described above in the preferred embodiment of step c) of bringing the homogenous supersaturated solution in contact with the seeding material i.e. the preferred stirring rate during cooling crystallization is also between 100rpm to 500 rpm, more preferably of between 200rpm to 400 rpm, even more preferably of between 320 to 360rpm.

In a further preferred embodiment of the process described herein step d) of obtaining the microparticles comprises at least one filtration and/or drying.

Surprisingly it was found that the composition comprising microparticles had reasonable filtration characteristic, given the small particle size distribution, with alpha-values comprised between 1e12 and 1e13, comparable with those obtained with particles of sizes between 30 and 100 µm (d10 and d90, respectively) together with reasonable flowing ability (cf. Table 1). Such an outcome was unexpected as it had been speculated that the small size of the microparticles may lead to a significant degree of clumping.

In general, a person skilled in the art is aware of the different methods for drying compositions comprising microparticles such as spray drying or spray granulation.

When using spray drying, the composition comprising microparticles is introduced into a spray dryer and subjected to the drying process normally used in this apparatus. Optionally, also micrometer sized carrier particles like lactose or others can additionally be used during the drying process as it is usually done in solvent based drying processes and well described in the state of the art.

Moreover, what is described herein relates to the use of nanoparticles of a d90 between 10 nm and 999 nm as seeding material in cooling crystallization as it has been demonstrated for the first time that via employing nano-particles of a d90 between 10 nm and 999 nm as seeding material leads to reproducible and controllable formation of microparticles comparable to microparticles produced using cooling crystallization with microparticles as seeding material but omitting the step of micronization. Hence this new use has the advantage that all drawbacks of micronization are overcome.

### FIGURES

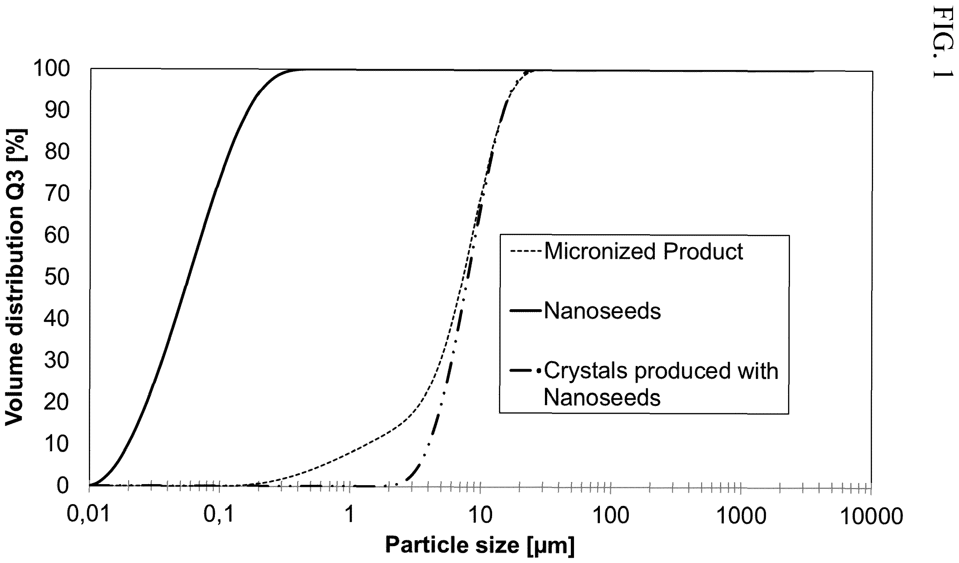

FIG. 1 shows that using nano seeding material (here active ingredient A) characterized by a nano-particle size of a d90 between 10 nm and 999 nm leads to micro-particles comparable to micro-particles generated with conventional processes.

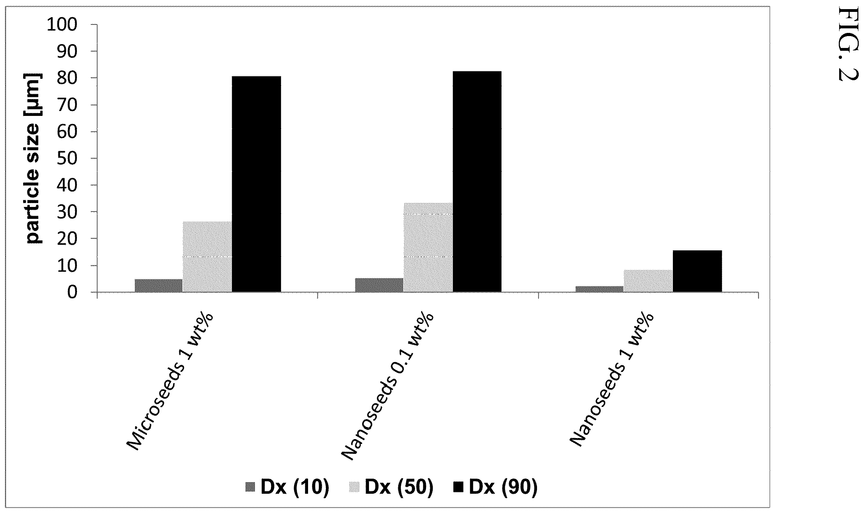

FIG. 2 depicts that when using nano seeding material (here active ingredient A) characterized by a nano-particle size of a d90 between 10 nm and 999 nm only 10% of the seeding material quantity is needed compared to using seeding material with a d90 above 999 nm in order to obtain a similar particle size distribution of the generated microparticles.

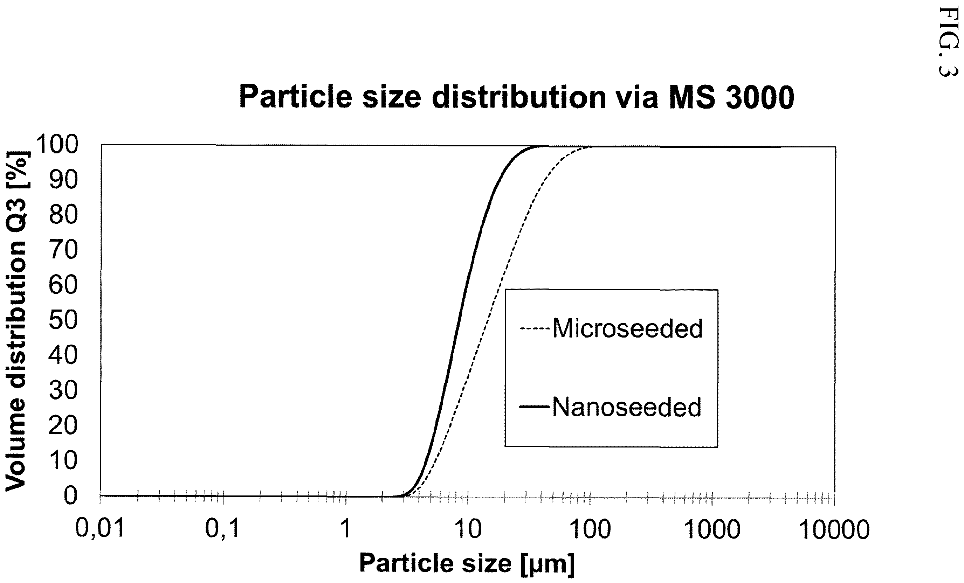

FIG. 3 shows that using nano seeding material (here active ingredient B) characterized by a nano-particle size of a d90 between 10 nm and 999 nm leads to micro particle typically obtained via conventional microseeding with subsequent micronization.

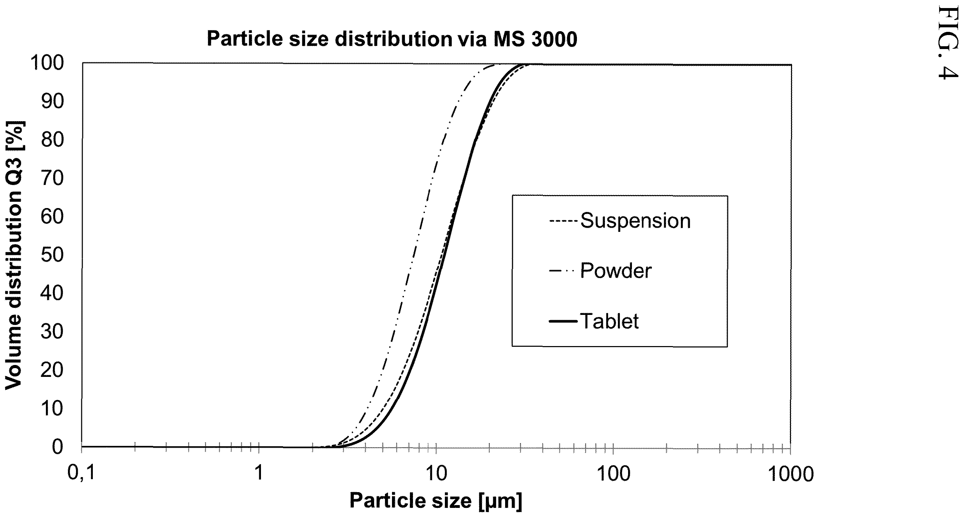

FIG. 4 shows that the nano seeding material can be stored as suspension, powder or as tablet and the microparticle generated with any of the three seeding material variants is comparable to the microparticles generated via conventional microseeding with subsequent micronization. When using powder generated according to example 1.2 below as seeding material the smallest microparticles were obtained.

### EXAMPLES

### 1. Generation of seeding material

### 1.1 Seeding material prepared in suspended state / Active ingredient A

In this example the seeding material was obtained via nano-grinding of 10 wt% active ingredient microparticles in 87 wt-% water while adding 3 wt% HPMC and 0.1 wt% SDS in a Bühler PML1 (small milling chamber) with a bead size of 0.1 mm (Yttrium stabilized Zirconium Oxide) for 40 min at 3000 rpm and a maximum temperature of 35°C thereby obtaining suspended nano-particles.

### 1.2 Powder or tablet seeding material obtained by the method described in WO 2021/069350 /Active Ingredient A

In one example the seeding material was obtained via nano-grinding of 10 wt% active ingredient microparticles in 79.8 wt-% water while adding 8 wt% PVP K12 and 2 wt% HPC and 0.2 wt% SDS in a Bühler PML1 (small milling chamber) with a bead size of 0.1 mm (Yttrium stabilized Zirconium Oxide) for 40 min at 3000 rpm and a maximum temperature of 35°C thereby obtaining suspended nano-particles.

In the next step the suspension was spray dried in a ProCept Lab Spray Dryer with a nozzle diameter of 0.8 mm, a drying inlet temperature of T = 110 °C, an atomization pressure of 0.4 bar at a volume flow of 0.3 m³/min and a feed flow of 5 g/min. The obtained powder was fully re-dispersible and part of the obtained powder was used as seeding material in the experiments described below. The rest of the obtained powder on the other hand was pressed to 150 mg tablets in a tablet press (StyleOne Medelpharm) at a tableting pressure of 100 MPa resulting in fully re-dispersible tablets. The powder and the tablet have the advantage, that they can be stored more easily and more stable that the material of 1.1. above.

### 1.3 Seeding material as powder / Active ingredient B

In a further example a different active ingredient was used. Also in this example the seeding material was obtained via nano-grinding of 10 wt% active ingredient microparticles in 79.8 wt-% water while adding 10 wt% PVP K12 0.2 wt% SDS in a Bühler PML1 (small milling chamber) with a bead size of 0.1 mm (Yttrium stabilized Zirconium Oxide) for 40 min at 3000 rpm and a maximum temperature of 35°C thereby obtaining suspended nano-particles.

In the next step the suspension was spray dried in a ProCept Lab Spray Dryer with a nozzle diameter of 0.8 mm, a drying inlet temperature of T = 110 °C, an atomization pressure of 0.4 bar at a volume flow of 0.3 m³/min and a feed flow of 5 g/min.

The obtained powder was fully redispersible and part of it was used as seeding material while the rest was pressed to tablets as described above under item 1.2 resulting in fully redispersible tablets.

### 2. Crystallisation

### 2.1 Active Ingredient (A)

The seeding material obtained in example 1.1 was analyzed via Laser diffraction (MS3000 by Malvern) and was determined to have a d90 of 250 nm. The material was used within 12 hours from its production. During the intervening time, the seeding suspension was hold unstirred in a refrigerated environment (2°C).

Then 1 wt-% with respect to total dissolved amount active ingredient of the seeding material obtained in example 1.1. was added to a vessel stirred by a curved impeller at 92°C and a stirring rate of 350 rpm containing 100 ml of a homogenous supersaturated solution of the same active ingredient in the crystallization medium pure acetic acid. After seeding the system was left ripening for 30 min at constant temperature of 92 °C. Afterwards the system was cooled in 3h at a constant cooling rate until 21°C were reached. A sample of the crystallized active ingredient was analyzed via laser diffraction and optical microscopy.

### 2.2. Active Ingredient B (Indomethacin)

The seeding material obtained in example 1.3 was analyzed via Laser diffraction (MS3000 by Malvern) and was determined to have a d90 of 250 nm.

Then 1 wt-% (with respect to total dissolved amount active ingredient in a homogenous supersaturated solution) of the seeding material (powder) obtained in example 1.3. was added to a vessel stirred by a curved impeller at 45°C and a stirring rate of 350 rpm containing a 50 ml homogenous supersaturated solution of the same active ingredient in the crystallization medium pure ethanol. After seeding the system was left ripening for 30 min at constant temperature of 45 °C. Afterwards the system was cooled in 3h at a constant cooling rate until 5°C were reached. Thereafter the system was left another 20 h at constant temperature of 5 °C. A sample of the crystallized active ingredient B was analyzed via laser diffraction and optical microscopy.

### 3. Filtration

### 3.1 Active Ingredient A

Following crystallization as described in example 2.1 the composition comprising micrometer sized particles suspended in acetic acid was filtered through a fine-pored filter cloth in a pressure nutsch at room temperature. Thereafter rinsing with acetic acid 1:1 and then with water 6:1 at a pressure of 1 bar took place. The three filtration steps were examined and resulted in specific filter cake resistances of 4x1012, 8x1012 and 1x1013 which was an indicator for a good filterability.

### 3.2. Active Ingredient B

The suspension of active ingredient B obtained in example 2.1 was filtered in a common lab vacuum nutsch at room temperature using a fine-pored filter membrane without rinsing afterwards.

### 4. Drying

### 4.1 Active Ingredient A

Following filtration as described in example 3.1 the composition comprising active ingredient A micro-particles was dried in a Vacuum Mixer Dryer [Amixon VMT1] at 60 °C and a pressure < 30 mbar for 3 hours.

### 4.2. Active Ingredient B

Following filtration as described in example 3.2 the composition comprising active ingredient B micro-particles was dried in a vacuum drying chamber at 30 °C and a pressure < 30 mbar over night.

### 5 Characterization of the generated active ingredient micro-particles

### 5.1 Active ingredient A micro-particles

The resulting micro- particles showed a comparable particle size distribution as conventionally obtained micro-particles (cf. Fig. 1) as determined by laser diffraction (MS3000 by Malvern, Hydro MV, wet dispersed in deionized water with Tween80 as surfactant to improve wetting and 180 seconds of ultrasonic treatment).

In detail Fig. 1 shows that using nano seeding material characterized by a nano-particle size of a d90 between 10 nm and 999 nm (left curve - solid line "Nanoseeds") leads to micro-particles (curve "Crystals produced with Nanoseeds) comparable to micro-particles generated with conventional processes i.e. employing microparticles as seeding material and subsequent micronization of the particles to achieve the desired particle size distribution (dotted line curve "Micronized Product"). In this example the nano seeding material was obtained as described in point 1.1 above. Similar results were obtained with seeding material as described it item 1.2 above (data not shown).

**Table**

| | Micronized particles | Nanoseeds | Nanoseeds (2L scale) |
|---|---|---|---|
| Flowability value (ffc) | 1,7 | <2 | 2,2 |

Thus, using nano seeding material characterized by a nano-particle size of a d90 between 10 nm and 999 nm (termed "Nanoseeds" in Table 1 above) on 300 ml scale (i.e. the volume of the homogenous supersaturated solution in step a) was 300 ml) leads to microparticle powder with a comparable flowability to microparticle powder obtained via conventional microseeding and subsequent micronizing. Moreover, the same results was achieved at the lager 2L (i.e. the volume of the homogenous supersaturated solution in step a) was 2L) scale.

Furthermore, as demonstrated in Fig. 2 it was found that when using nano seeding material characterized by a nano-particle size of a d90 between 10 nm and 999 nm only 10% of the seeding material quantity is needed compared to using seeding material with a d90 above 999 nm in order to obtain a similar particle size distribution of the generated microparticles. Thus, using nano seeding material characterized by a nano-particle size of a d90 between 10 nm and 999 nm as seeding material is more cost effective as significantly less seeding material is needed to generate the desired particle size distribution. Also for this reason the method and use described herein are advantageous e.g. more cost-efficient. Without being bound by theory it is thought that as the nano seeding material provides for considerably more crystallization nuclei, a smaller overall quantity of the nano seeding material compared to conventional seeding material is required to obtain the same quantity of microparticles of the desired size. The seeding material was generated as described in example 1.1. above.

When using seeding material generated as described in example 1.2 above, it was found that in general the same results were obtain as with seeding material generated according to 1.1. but the using powder obtained according to 1.2 lead to even finer micrometer sized particles (cf. Fig. 4)

### 5.2 Characterization of the generated active ingredient B micro-particles

The characterization of the active ingredient B micro-particles showed that also with this ingredient using a nano-particle size of a d90 between 10 nm and 999 nm prepared as described above in example 1.3 resulted in micro particles comparable to micro-particles generated with conventional processes i.e. employing microparticles as seeding material and subsequent micronization of the particles to achieve the desired particle size distribution (cf. Fig. 3).

Overall, it was found that using the method described herein the micronization step could be omitted and less seeding material was necessary to obtain a micro-particle powder comparable to the micro-particle powder derived from the conventional process.

## Claims

1. Method for generating micro- particles comprising the steps of
a) providing a homogenous supersaturated solution of a substance in a crystallization medium
b) providing a seeding material **characterized by** a nano-particle size of a d90 between 10 nm and 999 nm comprising at least one stabilizer, wherein said seeding material is of the same substance as the substance of the homogenous supersaturated solution of step a)
c) bringing the homogenous supersaturated solution in contact with the seeding material,
d) optionally obtaining micro-particles.

2. Process according to claim 1 wherein the seeding material is provided suspended in the crystallization medium or is provided as seeding material obtained by the method described in WO 2021/069350.

3. Process according to claim 2 wherein the seeding material obtained by the method described in WO 2021/069350 is provided as powder or as tablet.

4. Process according to claim 1, wherein the substance provided as homogenous supersaturated solution is an active ingredient and hence the seeding material and any generated micro-particles are active ingredient particles.

5. Process according to claim 1, where after bringing the homogenous supersaturated solution in contact with the seeding material the supersaturated solution is cooled.

6. Process according to claim 1 wherein the step d) of obtaining the microparticles comprises at least one filtration and/or drying.

7. Use of nanoparticles of a d90 between 10 nm and 999 nm as seeding material in cooling crystallization.
